# EUROPÄISCHE PATENTSCHRIFT

(11) **EP 2 705 158 B1**
(45) Veröffentlichungstag und Bekanntmachung des Hinweises auf die Patenterteilung: **27.04.2016**
(21) Anmeldenummer: 12721758.6
(22) Anmeldetag: 27.04.2012
(51) Int. Cl.: C12Q 1/68

(54) **VERFAHREN ZUR DETEKTION VON CHROMOSOMENABERRATION**
METHOD FOR DETECTING A CHROMOSOMAL ABERRATION
PROCÉDÉ POUR LA DÉTECTION D'ABERRATIONS CHROMOSOMIQUES

(30) Priorität: 02.05.2011 DE 102011100242
(43) Veröffentlichungstag der Anmeldung: 12.03.2014
(73) Patentinhaber: ZytoVision GmbH, 27572 Bremerhaven (DE)
(72) Erfinder: HAUKE, Sven, 28203 Bremen (DE)
(74) Vertreter: Von Rohr Patentanwälte Partnerschaft mbB
(86) Internationale Anmeldenummer: PCT/EP2012/001825
(87) Internationale Veröffentlichungsnummer: WO 2012/150022

(56) Entgegenhaltungen:
- WO-A1-92/05185
- WO-A2-02/093130
- WO-A2-2005/111235
- WO-A2-2009/102446
- OSOEGAWA ATSUSHI ET AL: "Incidentally proven pulmonary "ALKoma".", INTERNAL MEDICINE (TOKYO, JAPAN) 2010 LNKD- PUBMED:20228600, Bd. 49, Nr. 6, 2010, Seiten 603-606, XP002680763, ISSN: 1349-7235
- BURG VAN DER M ET AL: "Split-signal FISH for detection of chromosome aberrations in acute lymphoblastic leukemia", LEUKEMIA, MACMILLAN PRESS LTD, US, Bd. 18, Nr. 5, 1. Mai 2004 (2004-05-01), Seiten 895-908, XP002359905, ISSN: 0887-6924, DOI: 10.1038/SJ.LEU.2403340 in der Anmeldung erwähnt
- BOOMER T ET AL: "Detection of E2A translocations in leukemias via fluorescence in situ hybridization.", LEUKEMIA : OFFICIAL JOURNAL OF THE LEUKEMIA SOCIETY OF AMERICA, LEUKEMIA RESEARCH FUND, U.K JAN 2001 LNKD- PUBMED:11243406, Bd. 15, Nr. 1, Januar 2001 (2001-01), Seiten 95-102, XP002680766, ISSN: 0887-6924 in der Anmeldung erwähnt
- HOPMAN A H ET AL: "Multi-colour brightfield in situ hybridisation on tissue sections.", HISTOCHEMISTRY AND CELL BIOLOGY 1997 OCT-NOV LNKD- PUBMED:9387920, Bd. 108, Nr. 4-5, Oktober 1997 (1997-10), Seiten 291-298, XP002680765, ISSN: 0948-6143 in der Anmeldung erwähnt

## Beschreibung

Die vorliegende Erfindung betrifft ein Verfahren zur Detektion mehrerer unterschiedlicher Chromosomen- oder DNS-Bereiche in einer Zelle zum Nachweis von strukturellen Chromosomenaberrationen, wobei die Chromosomenaberrationen mindestens zwei Bruchpunktbereiche innerhalb eines Chromosoms aufweisen, basierend auf direkt oder indirekt markierten Nukleinsäurefragmenten (Sonden) und eine für dieses Verfahren geeignete Zubereitung.

Vielen Tumorerkrankungen liegen strukturelle Chromosomenmutationen wie Translokationen, Inversionen und segmentelle Duplikationen zu Grunde. Der Nachweis dieser Veränderungen als prädiktiver, prognostischer oder differentialdiagnostischer Marker geschieht in der Regel durch In-Situ-Hybridisierungen (ISH) unter Verwendung von Fluoreszenz-markierten (Fluoreszente ISH (FISH)) Nukleinsäurefragmenten, sogenannten Sonden, oder Hapten-markierten Sonden, welche anschließend mit Antikörpern detektiert und durch Farbreaktionen lichtmikroskopisch (Hellfeld ISH (BrISH) - dazu zählen u.a. auch Chromogene ISH (CISH) und Silber ISH (SISH)) - sichtbar gemacht werden.

Der Vorteil der FISH ist hierbei, dass ohne Mehraufwand in der Durchführung der Methode multiple genomische Bereiche simultan, aber voneinander deutlich unterscheidbar, nachgewiesen werden können. Hierzu werden Nukleinsäurefragmente, die unterschiedliche genomische Bereiche adressieren, mit unterschiedlichen Fluoreszenzfarbstoffen, die sich in ihrem Absorptions- und/oder Emissionsspektrum voneinander unterscheiden, markiert, d.h. gekoppelt. Werden solche mehrfarbigen Sonden z. B. an Metaphase-Chromosomen-Präparaten oder an Interphase-Zellkern-Präparaten verwendet, so können die einzelnen Farben durch Verwendung spezifischer Mikroskop-Filter-Sets, die genau definierte Wellenlängenbereiche des Lichts zur Anregung der Farbstoffe auf das Präparat leiten sowie genau definierte Wellenlängenbereiche des von den Farbstoffen emittierten Lichts zum Auswerter durchlassen, getrennt voneinander dargestellt werden (sog. Single-Bandpass-Filterset) oder aber es werden die unterschiedlichen Fluoreszenzsignale mehrerer Fragmente (bei zwei unterschiedlichen Fluoreszenzfarbstoffen spricht man hierbei von einem Dual-Bandpass-Filterset) gleichzeitig dargestellt.

Der simultanen Darstellung sind jedoch deutliche Grenzen gesetzt, da die Absorptions- und Emissionsbereiche der Farbstoffe häufig so nah beieinander liegen, dass sie durch die Mikroskop-Filter-Sets nicht voneinander getrennt werden können. Außerdem führen zwei sich überlagernde Signale unterschiedlicher Farben zur Wahrnehmung von Mischfarben (z. B. ergeben rot (oder orange) und grün dabei gelb), die sich nicht unterscheiden lassen von gleichfarbigen Signalen, die das Resultat eines anderen Fluorochroms sind und nicht durch Überlappung entstanden. Aus diesen Gründen werden in der Routine-FISH simultan zumeist nur zwei Farben (orange/rot und grün) bzw. drei Farben (orange/rot und grün simultan mit einer blauen Kerngegenfärbung (DAPI)) betrachtet. In etwa die gleichen Einschränkungen, die für die FISH darstellbar sind, gelten für die BrISH. Hier ist der Stand der Technik die Verwendung von zwei Haptenen, zumeist ausgewählt aus der Gruppe Biotin, Dinitrophenyl (DNP) und Digoxigenin, und zwei antikörpergekoppelten Enzymen, zumeist alkalische Phosphatase und Peroxidase (Carbone et al., J. Mol. Diagn. 2008, 10(6):527-536; Hopman et al., Histochem. Cell Biol. 1997, 108(4-5):291-298; Laakso et al., J. Pathol. 2006, 210(1):3-9; Mayr et al., Histopathology 2009, 55(6):716-723; Tanner et al., Am. J. Pathol. 2000, 157(5):1467-1472).

Die genannten Einschränkungen bei der simultanen Darstellung haben einen maßgeblichen Einfluss auf die Kompositionen der Sonden für den Nachweis von Translokationen und Inversionen. Hier gibt es prinzipiell zwei maßgebliche Techniken und zugrunde liegende Sondenkompositionen: Das Prinzip des Entstehens von Fusionssignalen (sog. Dual-Color-Dual-Fusion-Ansätze) (WO02093130, Dewald et al., Blood 1998, 91(9):3357-3365; Dewald et al., Cancer Genet. Cytogenet. 2000, 116(2):97-104; Wan et al., J. Clin. Pathol. 2003, 56(6):471-474) bzw. des sich Auftrennens von Fusionssignalen (sog. Dual-Color-Break-Apart- bzw. Dual-Color-Split-Ansätze (van der Burg et al., Leukemia 1999, 13(12):2107-2113; Boomer et al., Leukamia 2001, 15(1):95-102; van der Burg et al., Leukemia 2004, 18(5):895-908). In der nachfolgenden Darstellung dieser beiden Prinzipien und abgeleiteten Signalmuster ist zu beachten, dass eine normale Zelle in der Regel diploid ist, d. h. jedes Allel zweifach vorliegt. Da von Aberrationen in der Regel jeweils nur eines der beiden Allele betroffen wird, ist neben dem aberranten Signal in der Regel auch noch das normale Signal des nicht von der Aberration betroffenen Allels sichtbar. Zum besseren Verständnis wird nachfolgend das Signalmuster des normalen Signals nicht immer explizit beschrieben.

Bei Dual-Color-Dual-Fusion-Ansätzen wird der Bereich des Bruchpunkts 1 proximal und distal von Nukleinsäurefragmenten der gleichen Farbe (z. B. orange) flankiert, der Bereich des Bruchpunktes 2, d. h. des reziproken Translokationspartners, wird proximal und distal von Nukleinsäurefragmenten einer zweiten Farbe (z. B. grün) flankiert. Die normale Situation, d. h. ohne chromosomale Brüche im Bereich der beiden Translokationspartner, wird hierbei durch ein grünes und ein räumlich getrenntes oranges Signal charakterisiert.

Im Falle einer reziproken Translokation kommt es innerhalb der Bruchpunkte beider Translokationspartner zu Brüchen und der proximale Bereich des einen Translokationspartners fusioniert mit dem distalen Bereich des anderen Partners und vice versa. Es entstehen somit zwei grün/orange Signal-Paare, auch Fusionssignale genannt, da sich die unterschiedlich farbigen Signale oftmals überlappen, so dass ein Mischfarbenes-Signal sichtbar ist. Der Nachteil dieser Sonden-Techniken ist, dass die Fusionssignale nur entstehen, wenn die Bruchpunkte beider Translokationspartner im Bereich der jeweiligen markierten Nukleinsäurefragmente liegen. Bei Translokationen, die nur einen der beiden Partner betreffen, entstehen keine Fusionssignale. Es kommt hierbei lediglich zur Entstehung eines zusätzlichen Signals mit der Farbe des Signals, welches charakteristisch für den durch die Translokation betroffenen Partner ist. D. h., es entsteht z. B. ein zusätzliches grünes Signal wenn der Bruchpunkt der Translokation in dem Bereich lag, der von den mit grünem Fluorochrom markierten Nukleinsäuren überdeckt wurde. Solche Zusatzsignale können jedoch häufig durch andere Zellkerne überdeckt werden, dicht bei einem der anderen gleichfarbigen Signale liegen und somit nicht als Zusatzsignal wahrgenommen werden, oder durch Gewebsschnittartefakte verloren gehen. In diesem Fall kann es zur Falschdiagnose kommen, da eine nur den einen Translokationspartner betreffende Translokation nicht wahrgenommen wird.

Bei Dual-Color-Break-Apart-Ansätzen wird der Bereich eines Bruchpunktes proximal und distal von unterschiedlich markierten oder farbig-markierten Nukleinsäurefragmenten flankiert (z. B. distal orange, proximal grün). Die normale Situation, d. h. ohne chromosomalen Bruch dieses Bereiches, wird dabei durch ein Fusionssignal charakterisiert. In einer aberranten Situation, d. h. wenn es zu einem chromosomalen Bruch zwischen den Sondenfragmenten kommt, trennen sich die Signale räumlich voneinander. Der Unterschied zwischen der normalen Situation und der aberranten Situation ist also gekennzeichnet durch den Abstand der unterschiedlich farbigen Signale.

Der Nachteil dieser Sonden-Kompositionen ist, dass Aussagen über den beteiligten Translokationspartner nicht möglich sind. Außerdem kann es, bedingt z. B. durch die Sichtachse mit der der Betrachter auf den Interphasezellkern sieht, dazu kommen, dass zwei räumlich getrennte Signale, die in Bezug auf die Sichtachse übereinander liegen, weiterhin als Fusionssignal wahrgenommen werden.

Neben den o. g. Zweifarb-Anwendungen werden in FISH-Analysen auch Dreifarb-, Vierfarb- und Fünffarbsonden verwendet. Dabei werden neben den deutlich stärkeren Standard-Fluoreszenzfarben orange/rot und grün die weiteren, zur Verfügung stehenden schwächeren Farben, z. B. gold oder rot oder blau verwendet. Daher werden in Routineanwendungen beispielweise Vierfarb-FISH-Sonden nur zur Detektion von Deletionen oder Amplifikationen verwendet (EP1035215B1, WO2007/028031, EP0549709B1), weil hier zumeist bei den Markierungen der Sonden auf repetitive Sequenzen zur Verstärkung der Farbintensitäten z. B. von blau und gold zurückgegriffen werden kann.

Die von Fink et al. (Leuk. Res. 2009, 33(6):843-846) beschriebenen Dreifach-FISH-Ansätze adressieren den Nachweis von unterschiedlichen Translokationsereignissen, die in einer chromosomalen Region nebeneinander clustern können (d. h. es sind unterschiedliche Gene betroffen, die in Nachbarschaft liegen). Dabei werden für die entsprechende Auswertung der Signalmuster jeweils nur zwei Farben betrachtet, die eine einzelne Aberration nachweisen, wobei die dritte Farbe dann jeweils keine Rolle spielt.

Betreffend BrISH unter Verwendung von mehr als zwei Farben beschreiben Hopman et al. (Histochem. Cell Biol. 1997, 108(4-5):291-298) die Durchführung einer chromogenen Dreifach-In-situ-Hybridisierung, die allgemein auf den Nachweis von drei repetitiven chromosomalen Bereichen abzielt. Nach dem derzeitigen Stand der Technik werden Translokationen mittels BrISH nur unter Verwendung von zwei Haptenen und somit zwei Farbstoffen nachgewiesen.

Ebenfalls unbekannt ist die Verwendung der BrISH-Methode für den Nachweis von Inversionen und Insertionen.

Inversionen und insbesondere kleine Inversionen können nicht mit den Techniken und Verfahren, die in den Patentanmeldungen WO02093130A3 und WO 2005/111235 A2 beschrieben sind, detektiert werden. Die WO 2005/111235 A2 beschreibt ein Verfahren, das die Verwendung von drei Farben Sonden aufweist. Jedoch wird die chromosomale Region, die von dem 3. Label einer Sonde markiert wird, nicht unmittelbar von einer Veränderung betroffen, so dass bei einer Chromosomenstrukturänderung das erste Fusionssignal eliminiert wird, dabei entsteht ein neues Split Signal und ein neues Fusionssignal. Dieses Ereignis kann bei Inversionen, insbesondere kleinen Inversionen, nicht oder nur sehr schlecht von einer normalen Situation unterschieden werden.

Die WO02093130A3 offenbart ein Verfahren zur Detektion von chromosomalen Translokationen unter Verwendung von zwei bzw. vier Label/Farbstoffen, dabei werden die Sonden auf unterschiedlichen Chromosomen hybridisiert. Dieses Verfahren eignet sich nur bedingt für den Nachweis von Inversionen. Zudem ist das Auswerteverfahren aufgrund der Vielzahl der Signale, die sich aus vier Label/Farbstoffen ableiten lassen, deutlich komplizierter.

Weiterhin betrifft die WO 2009/102446 A2 Verfahren und Zusammensetzungen speziell zur Detektion von chromosomalen Inversionen zwischen EML4 und ALK mittels FISH, wobei die darin beschriebene Fluoreszenz-in-situ-Hybridisierung insbesondere für diagnostische und prognostische Zwecke sowie für Therapie-Strategien eingesetzt werden kann. Für das Detektionsverfahren werden lediglich zwei Sonden für einen Dual-Color-Single-Fusion-Ansatz eingesetzt.

Zudem beschreiben Osoegawa Atsushi et al. im Rahmen der wissenschaftlichen Publikation "Incidentally proven pulmonary ALKoma", erschienen in Intern. Med. 2010, ein Verfahren zur Detektion von EML4-ALK Inversionen mittels FISH, welches auf einem Split-Signal-Ansatz bzw. Dual-Color-Break-Apart-Ansatz mit zwei Sonden basiert.

Alle derzeit verfügbaren Methoden und Sondenkompositionen für BrISH und FISH im Zusammenhang mit Inversionen, insbesondere, aber nicht darauf beschränkt, bei Tumoren, haben Mängel. Es gibt keine Sondenkompositionen und Methoden und deren entstehende Signalmuster, die in jedem Fall die sichere Erkennung einer Inversion erlauben. Insbesondere bei kleinen Inversionen d. h. Inversionen kurzer genomischer Abschnitte (beispielsweise Bereiche kleiner als 20 Mb) wird dieses Aberrationsereignis zumeist nicht erkannt.

Aufgabe der Erfindung ist es daher ein Verfahren bereitzustellen, mit dem ein sicherer Nachweis von strukturellen Chromosomenaberrationen, wobei die Chromosomenaberrationen mindestens zwei Bruchpunktbereiche innerhalb eines Chromosoms aufweisen, ermöglicht wird.

Zur Lösung der Aufgabe wird ein Verfahren der Eingangs genannten Art vorgeschlagen, wobei
- eine mit einem Label A markierte erste Sonde (Sonde A) und eine mit einem Label B markierte zweite Sonde (Sonde B) einen Bruchpunktbereich 1 flankieren, die Fusionssignale A-B bilden und
- zwei mit jeweils einem Label C markierten dritte und vierte Sonde (Sonden C) einen Bruchpunktbereich 2 flankieren, die Fusionssignale C-C bilden, wobei die vorgenannten Fusionssignale sich bei einer Chromosomenaberration zu Fusionssignale A-C und Fusionssignale B-C verändern.

Bruchpunktbereich 1 und Bruchpunktbereich 2 beschreiben mögliche Bruchpunkte innerhalb eines Chromosoms.

Der Begriff "flankieren" wird hierbei derart verstanden, dass Sonde A und Sonde B jeweils auf eine Seite des Bruchpunktbereichs 1 und analog die Sonden C jeweils auf eine Seite des Bruchpunktbereichs 2 hybridisieren.

Es ist ebenfalls vorstellbar, eine einzige Sonde C über den Bruchpunktbereich 2 zu überspannen, um den gleichen Effekt, nämlich der Bildung der Fusionssignale C-C, zu erhalten.

Unter Sonden werden Nukleinsäurefragmente verstanden, die mit einem bestimmten Label markiert sind.

Das erfindungsgemäße Verfahren erlaubt gegenüber den o. g. bekannten Methoden und Kompositionen erstmals einen eindeutigen und/oder überhaupt erstmals möglichen Nachweis von Inversionen, insbesondere auch kleineren, genomischen Inversionen, sowie auch die eindeutige Unterscheidung zu Translokationen.

Vorzugsweise handelt es sich bei den markierten Nukleinsäurefragmenten (Sonden) um Polynukleotide, modifizierte Polynukleotide oder modifizierte Nukleinsäurefragmenten oder Oligonukleotide oder modifizierte Oligonukleotide.

Beispielsweise sind dem Fachmann PNA und LNA für modifizierte Polynukleotide bekannt.

Bevorzugt weisen die Nukleinsäurefragmente unterschiedliche Label A, B und C auf, somit werden auch unterschiedliche Sonden A, B und C gebildet.

Hierzu wird eine Kombination aus drei unterschiedlichen Fluoreszenzfarbstoffen in der FISH oder drei unterschiedlichen Reportermolekülen in der BrISH für die Markierung der Nukleinsäurefragmente (oder allgemein Polynukleotide oder aber auch Oligonukleotide) verwendet.

Vorzugsweise wird das Label ausgewählt aus der Gruppe Farbstoffe, Farbstoffsubstrate, Chemielumineszenzfarbstoffe (z. B. Acridinium), Radioisotope, Spin-Label, Enzyme (z. B. Alkalische Phosphatase, Meerrettich-Peroxidase, Sojabohnen-Peroxidase und/oder beta-Galactosidase), Haptene (z. B. Dioxigenin, Biotin, 5(6)-Carboxyfluorescein, Rhodamin, Bromdeoxyuridin, Acetylaminofluoren, Trinitrophenol, Trinitrophenol -Derivat, Estradiol, und/oder DNP), Quantum Dots, Beads, Aminohexyle, Pyrene und Fluoreszenzfarbstoffe (z. B. Fluorecein, Fluorescein-Derivat, 5(6)-Carboxyfluorescein, Coumarin, Coumarin-Derivat, Rhodamin, Rhodamin-Derivat, Tetramethylrhodamin, Lissamin, Texas Red, AMCA, TRITC, IR Farbstoff, Alexa-Farbstoff, Dyomics-Farbstoff, Phycoerythrine, Cascade Blue, Oregon Green 488, Pacific Blue und/oder Rhodamine Green).

Das erfindungsgemäße Verfahren kann bevorzugt mittels der FISH-Methode unter Verwendung von drei direkt eingebauten Fluoreszenzfarbstoffen für die Emissionsbereiche Grün, Orange/Rot und Blau durchgeführt werden.

Auch mittels der FISH-Methode unter Verwendung von drei direkt eingebauten Fluoreszenzfarbstoffen für die Emissionsbereiche Grün, Rot und Gold ist das Verfahren durchführbar.

Die Auswertung erfolgt vorzugsweise durch Analyse von Fluoreszenz-Signalen.

Vorzugsweise wird das erfindungsgemäße Verfahren mittels der BrISH-Methode unter Verwendung von Biotin, Digoxigenin und DNP, welche mit Antikörper-gekoppelter alkalischer Phosphastase, Antikörper-gekoppelter Peroxidase und Antikörper-gekoppelter beta-Galactosidase verbinden, durchgeführt wird.

Überraschenderweise wurde festgestellt, dass bei dem erfindungsgemäßen Verfahren neue Fusionssignale A-C und B-C entstehen, wobei die Fusionssignale A-C bzw. B-C über ein zwei-Farben-Split-Ereignis der Fusionssignale A-B und über ein Einfarb-Split-Ereignis der Fusionssignale C-C gebildet werden. Als dadurch entstehendes Signalmuster (unter gleichzeitiger Verwendung/Betrachtung/Auswertung der dritten Farbe/des dritten Labels), können hierbei zwei neue Fusionssignale betrachtet werden. Das erfindungsgemäße Verfahren erlaubt somit erstmals und überraschend die o. g. Nachweise der o. g. Aberrationen, die mit dem Stand der Technik nicht oder nur sehr unzureichend möglich sind. Dabei ermöglicht eine gleichzeitige Auswertung der dritten Farbe/des dritten Labels neben den anderen beiden Farben/Label die erstmalige oder eindeutige Erkennung der Aberration.

Auch im Falle von genomischen Inversionen, insbesondere, aber nicht darauf beschränkt, kleineren Inversionen mit genomischen Abschnitten, die von der Inversion betroffen sind, von kleiner 20 Mb, kann die räumliche Trennung der Signale so gering ausfallen, dass sie weiterhin als Fusionssignal wahrgenommen werden. In diesen Fällen kann es zur Falschdiagnose kommen, da eine Translokation nicht als solche wahrgenommen wird.

Das erfindungsgemäße Verfahren ist bevorzugt für den Nachweis von Inversionen und/oder Insertion und/oder Duplikation geeignet.

Vorzugsweise zum Nachweis von Inversionen, Insertionen und/oder Duplikation kurzer genomischer Abschnitte.

Besonders bevorzugt liegen die invertierten, inserierten und/oder duplizierten genomische Abschnitte im Bereich kleiner als 20 Mb, bevorzugt kleiner als 15 Mb, besonders bevorzugt kleiner als 10 Mb und noch bevorzugter kleiner als 5 Mb.

Bei Inversionen kurzer genomischer Abschnitte entstehen zwei neu zusammengesetzte Fusionssignale, die beispielsweise bei einer FISH-Analyse in der Interphase mittels üblichen geeigneten Filtersets deutlich zu erkennen sind. Es ist jedoch nicht auszuschließen, dass bei größeren Bruchpunktbereichen die Fusionssignale A-C oder B-C räumlich auseinander liegen, so dass die Signale nicht mehr eindeutig als Fusionssignale zu erkennen sind. Zumindest liegt in diesen Fällen dann ein Signal C gesondert vor.

Es ist ebenfalls möglich, mithilfe des erfindungsgemäßen Verfahrens Inversionen von Translokationen zu unterscheiden.

Bevorzugt wird das erfindungsgemäße Verfahren zum Nachweis von Inversionen, wobei die Inversion inv(2)(p22-p21p23) und/oder die Gene ALK und/oder EML4 betroffen sind, eingesetzt.

In der FISH können dabei grundsätzlich mindestens zwei der Fluoreszenzfarbstoffe simultan durch ein entsprechendes Filter-Set wahrnehmbar sein, was ein schnelles und einfaches Screening analog der o. g. zweifarbigen Sondenansätze erlaubt, darüber hinaus jedoch eine zusätzliche diagnostische Sicherheit bietet.

Im Folgenden werden die Vorteile eines genomischen Inversions-Nachweises mittels der neuen Sondenkompositionen und der neuen ISH-Techniken in weiteren Details beschrieben:
Der sichere oder eindeutige Nachweis der Inversion kurzer genomischer Abschnitte (Bereiche kleiner als 20 Mb) ist mit herkömmlichen Sondenkompositionen nicht möglich. Zudem ist auch der Nachweis der Inversion größerer genomischer Abschnitte (Bereiche größer als 20 Mb) häufig nur sehr schwer möglich. Stand der Technik sind hierbei verschiedene Sondenkonzepte. Im einfachsten Fall wird nur einer der beiden Bruchpunktbereiche mit einem Dual-Color-Break-Apart-Ansatz betrachtet. Im Falle einer Inversion kommt es dabei zu einer räumlichen Trennung der Fluoreszenzsignale. Diese Signaltrennung tritt jedoch auch bei herkömmlichen reziproken Translokationen unter Involvierung des Bruchpunktbereiches auf, von denen eine Unterscheidung mit diesem Sonden-Konzept nicht möglich ist.

Ein weiterer Nachteil ist, dass bei dual colour break Apart-Sonden und -Techniken häufig auch im nicht-rearrangierten Zustand eine Signalauftrennung des Fusionssignals in dicht beieinander liegende aber getrennt voneinander wahrnehmbare Fluoreszenzsignale geschieht. Ist der genomische Abschnitt der Inversion per se nur sehr kurz (z. B. kleiner als 20 Mb), kann dies dazu führen, dass die zu erwartende räumliche Signaltrennung nur unwesentlich größer ist und somit übersehen werden kann. Eine Unterscheidung zwischen Inversion und reziproker Translokation ist mit diesem Ansatz somit nicht möglich, außerdem können aberrante Situationen als nicht-rearrangiert missinterpretiert werden.

Im Falle eines Dual-Color-Dual-Fusion-Ansatzes würde der proximale Bruchpunktbereich mit z. B. grün markierten Nukleinsäurefragmenten und der distale Bruchpunktbereich mit z. B. orange markierten Nukleinsäurefragmenten überspannt. Im Falle der Inversion eines kurzen genomischen Abschnitts, z. B. kleiner als 20 Mb, entstünden zwei sehr nahe beieinander liegende grüne Signale und ebenfalls zwei sehr nahe beieinander liegende orange Signale. Bei Signalüberlagerung entstünden somit zwei sehr nahe beieinander liegende Fusionssignale, die auf Grund ihrer räumlichen Nähe, beispielsweise auch mit einem entsprechenden Dual-Bandpass-Filterset, häufig nicht sicher als zwei getrennte Fusionssignale wahrgenommen werden könnten. Abhilfe könnte hierbei beispielsweise auch die Verwendung von Single-Bandpass-Filtersets spezifisch für die beiden Signalfarben schaffen. Dies würde den Auswerteprozess jedoch verlangsamen und verkomplizieren. Des Weiteren kommt es bei Abschnitten dekondensierten Chromatins auch bei normalen Allelen häufig zur Entstehung perlenschnurartiger, länglicher Signale, von denen sich zwei, auf Grund einer Inversion, räumlich getrennte Signale nicht zwangsweise unterscheiden lassen. Die Missinterpretation zweier nahe beieinander liegender Fusionssignale als nur ein Fusionssignal würde zu einer falsch-negativen diagnostischen Aussage führen.

Die Sondenkomposition der vorliegenden Erfindung kombiniert u. a. auch den Dual-Color-Break-Apart-Ansatz für den Nachweis des Bruchs in einem der beiden Bruchpunktbereiche mit einem Single-Color-Break-Apart-Ansatz unter Verwendung eines dritten Labels, z.B.Fluorochroms in der FISH, z.B. blau, welches getrennt von den beiden ersten Labeln, z.B. Farbstoffe (z.B. grün und orange) nachgewiesen werden kann, für den Nachweis des Bruchs im zweiten Bruchpunktbereich. Im Falle einer Inversion kommt es dabei zu einer, beispielsweise bei einer FISH-Anwendung im Dual-Bandpass-Filterset in der Regel wahrnehmbaren, Auftrennung der Fusionssignale in ein räumlich getrenntes grünes und oranges Signal. Gleichzeitig kommt es beispielsweise, wahrnehmbar in einem zweiten Filterset, spezifisch für blau, durch den Bruch in der zweiten Bruchpunktregion zu einer Auftrennung des blauen Signals in zwei räumlich getrennte Signale. Im Gegensatz zu den herkömmlichen Ansätzen wird eine Inversion also durch zwei unterschiedlich voneinander wahrnehmbare Ereignisse charakterisiert, was zu einer deutlich sichereren diagnostischen Aussage, gerade bei unklaren Signalmustern führt. So kann im Falle einer unklaren grün-orangen-Signaltrennung eine Absicherung über die Auswertung im Single-Bandpass-Filterset für das blaue Signal erfolgen. Kommt es auch hier zu einem zusätzlichen blauen Signal und ko-lokalisieren jeweils ein blaues Signal jeweils mit einem grünen und einem orangen Signal, so gilt dies als Bestätigung der Inversion. Liegt jedoch ein normales blaues Signalmuster vor, so gilt das fragliche grün-orange-Signal ebenfalls als nicht-aberrant. Im Falle einer unzweideutigen Signalauftrennung in ein grünes und ein oranges Signal dient die Auswertung des blauen Signalmusters zur Unterscheidung zwischen einer Inversion, in diesem Fall liegt ein zusätzliches blaues Signal vor, und einer Translokation mit unbekanntem Translokationspartner, in diesem Fall liegt ein normales blaues Signalmuster vor.

Ein weiterer Gegenstand der Erfindung ist eine erfindungsgemäße Zubereitung zur Detektion mehrerer unterschiedlicher Chromosomen- oder DNS-Bereiche in einer Zelle zum Nachweis von strukturellen Chromosomenaberrationen, wobei die Chromosomenaberrationen mindestens zwei Bruchpunktbereiche auf einem Chromosomen aufweisen, basierend auf direkt oder indirekt markierten Nukleinsäurefragmenten (Sonden), umfasst
- eine mit einem Label A markierte erste Sonde (Sonde A) und eine mit einem Label B markierte zweite Sonde (Sonde B) einen Bruchpunktbereich 1 flankieren, die Fusionssignale A-B bilden und
- zwei mit jeweils einem Label C markierten dritte und vierte Sonde (Sonden C) einen Bruchpunktbereich 2 flankieren, die Fusionssignale C-C bilden, wobei die vorgenannten Fusionssignale sich bei einer Chromosomenaberration zu Fusionssignale A-C und Fusionssignale B-C verändern.

Vorzugsweise weist die erfindungsgemäße Zubereitung Sonden auf, die mit den oben genannten Labeln markiert sind.
Anhand der folgenden Zeichnungen und Beispielen wird die Erfindung näher beschrieben. Es zeigt:
- Fig. 1:: Schematische Darstellung eines erfindungsgemäßen Verfahrens für die Detektion einer Inversion
- Fig. 2:: Schema zu Signalmustern bei Verwendung einer Dreifach-FISH-Sonde "ZytoLight SPEC ALK/EML4 TriCheck™ Probe" der Fa. ZytoVision GmbH
- Fig. 3:: FISH-Analyse zum Nachweis der Inversion inv(2)(p22-p21p23 unter Verwendung der Dreifach-FISH-Sonde "ZytoLight SPEC ALK/EML4 TriCheck™ Probe"
- Fig. 4:: CISH-Analyse zum Nachweis der Inversion inv(2)(p22-p21p23 unter Verwendung der Dreifach-CISH-Sonde "ZytoDot 2C SPEC ALK/EML4 TriCheck™ Probe" der Fa. ZytoVision GmbH

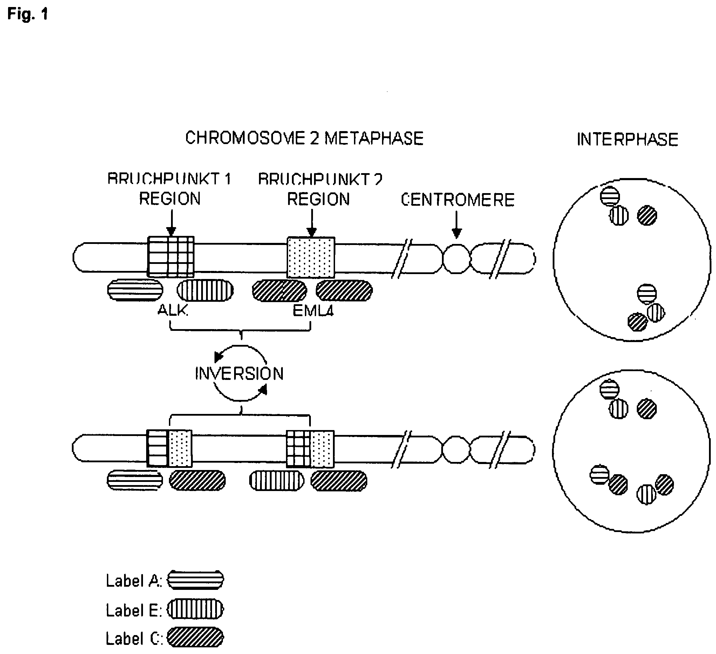

Fig. 1 zeigt eine schematische Darstellung eines erfindungsgemäßen Verfahrens für die Detektion einer Inversion unter Verwendung von drei Labeln. Es zeigt das Signalmuster bei der Inversion inv(2)(p22-p21 p23), die die Gene ALK und EML4 betrifft. Label A und B einer Dreifach-ISH-Sonde flankieren jeweils auf einer Seite der Bruchpunkt Region 1 (ALK) und Label C jeweils auf einer Seite der Bruchpunkt Region 2 (EML4).

In der Interphase einer normalen Zelle (ohne Aberrationen, oberer Teil der Figur) sind zwei Fusionssignale bestehend aus Label A und Label B und zwei Signale von Label C zu erkennen. In einer von einer Inversion betroffenen Interphase (unterer Teil der Figur) treten ein neues Fusionssignal A-C, bestehend aus Label A und Label C sowie ein neues Fusionssignal B-C bestehend aus Label B und Label C. Daneben sind Fusionssignale und Einzelsignale des normalen Chromosoms.

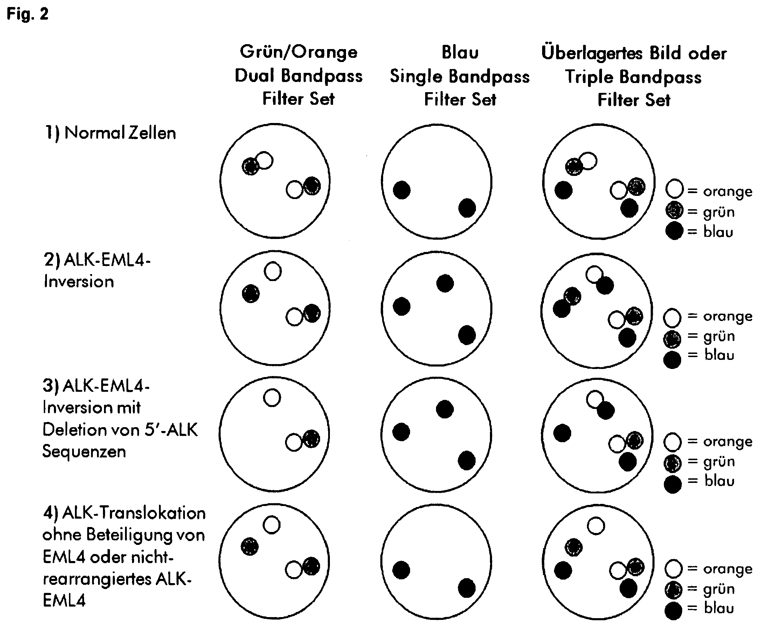

Fig. 2 zeigt ein Schema zu Signalmustern bei Verwendung einer Dreifach-FISH-Sonde "ZytoLight SPEC ALK/EML4 TriCheck™ Probe" der Fa. Zytovision. Die Sonde besteht aus grün-markierten Polynukleotiden (Absorption bei 503 nm und Emission bei 528 nm), die in 2p23 gegen proximal zur ALK-Bruchpunktregion gelegene Sequenzen gerichtet sind, orange-markierten Polynukleotiden (Absorption bei 547 nm und Emission bei 572 nm), die in 2p23 gegen distal zur ALK-Bruchpunktregion gelegene Sequenzen gerichtet sind, sowie blau-markierten Polynukleotiden (Absorption bei 426 nm und Emission bei 480 nm), die in der Region 2p21 distal und proximal zur EML4-Bruchpunktregion gelegene Sequenzen gerichtet sind.

Bei Verwendung geeigneter Filtersätze erscheinen die Hybridisierungssignale für das ALK-Gen (2p23) als grüne und orange Fluoreszenzsignale, die Hybridisierungssignale für das EML4-Gen (2p21) erscheinen als blaue Fluoreszenzsignale.

In normalen Zellen bzw. Zellen ohne ALK-EML4-Rearrangierungen erscheinen in der Interphase zwei grün/orange Fusions-Signale bei Verwendung eines geeigneten Dual-Bandpass-Filtersets und zwei blaue Signals bei Verwendung eines geeigneten Single-Bandpass-Filtersets (s. Fig. 1).

Ein von einer ALK-EML4-Inversion betroffener 2p21-23-Lokus ist durch ein separates grünes und ein separates oranges Signal sowie ein zusätzliches blaues Signal gekennzeichnet. Dabei liegt das separate grüne und das orange Signal jeweils neben einem blauen Signal (s. Fig. 2).

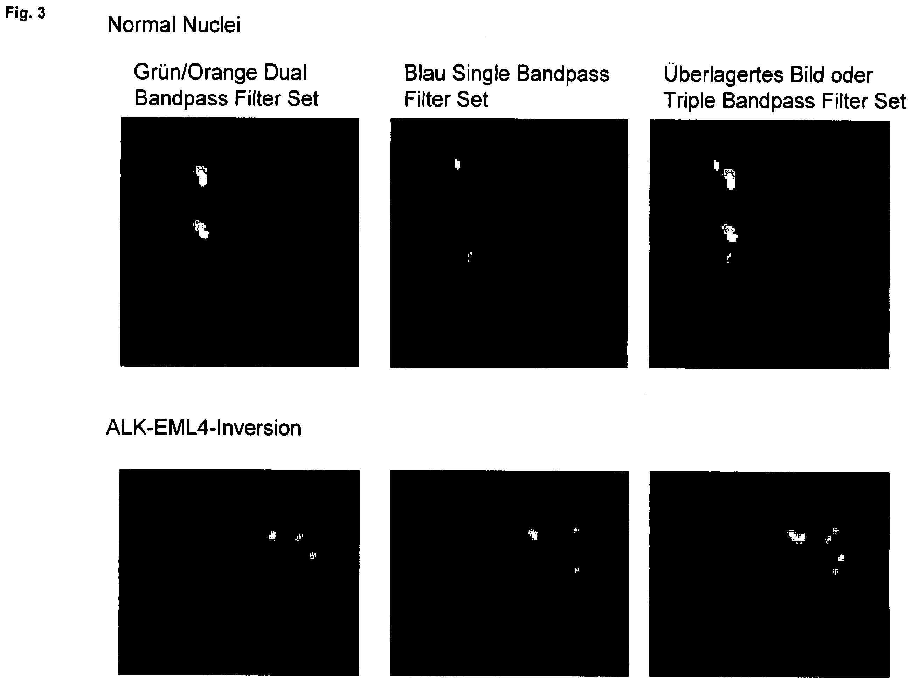

Ein von einer ALK-EML4-Inversion betroffener 2p21-23-Lokus mit einer Deletion von 5'-ALK-Sequenzen ist durch den Verlust eines grünen Signals und durch ein zusätzliches blaues Signal gekennzeichnet. Dabei liegt das orange Signal neben einem blauen Signal (s. Fig. 3).

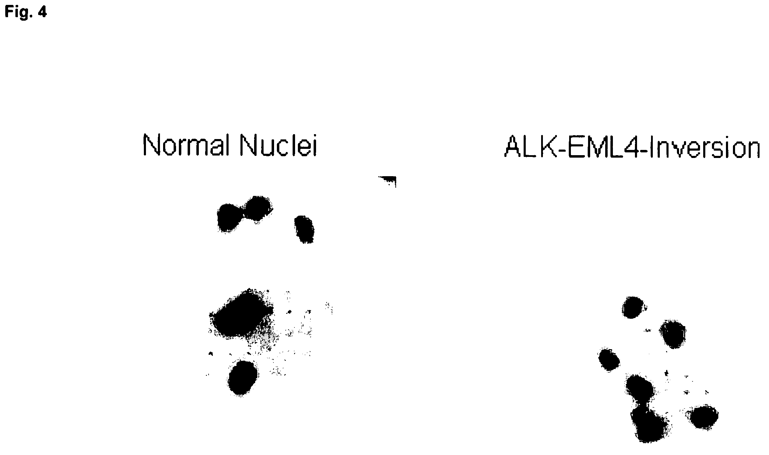

Ein separates grünes und ein separates oranges Signal in Kombination mit einer normalen Anzahl blauer Signale (s. Abb. 4) steht entweder für eine ALK-Translokation ohne Einbeziehung des EML4 oder, wenn das separate grüne und orange Signal sehr nahe bei einander liegen, für einen nicht rearrangierten 2p21-23-Lokus.

Fig. 3 zeigt eine FISH-Analyse zum Nachweis der Inversion inv(2)(p22-p21p23 unter Verwendung der Dreifach-FISH-Sonde "ZytoLight SPEC ALK/EM L4 TriCheck™ Probe". Die Sonde besteht aus grün-markierten Polynukleotiden (Absorption bei 503 nm und Emission bei 528 nm), die in 2p23 gegen proximal zur ALK-Bruchunktregion gelegene Sequenzen gerichtet sind, orange-markierten Polynukleotiden (Absorption bei 547 nm und Emission bei 572 nm), die in 2p23 gegen distal zur ALK-Bruchpunktregion gelegene Sequenzen gerichtet sind, sowie blau-markierten Polynukleotiden (Absorption bei 426 nm und Emission bei 480 nm), die in der Region 2p21 distal und proximal zur EML4-Bruchpunktregion gelegene Sequenzen gerichtet sind. Mit geeigneten Filtersets lässt sich das Signalmuster gut sichtbar machen.

Fig. 4 zeigt eine CISH-Analyse zum Nachweis der Inversion inv(2)(p22-p21p23) unter Verwendung der Dreifach-CISH-Sonde "ZytoDot 2C SPEC ALK/EML4 Tri-Check™ Probe". Die Sonde besteht aus Digoxigenin-markierten Polynukleotiden Polynukleotiden, die in 2p23 gegen proximal zur ALK-Bruchpunktregion gelegene Sequenzen gerichtet sind, DNP-markierten Polynukleotiden, die in 2p23 gegen distal zur ALK-Bruchpunktregion gelegene Sequenzen gerichtet sind, sowie Biotinmarkierten Polynukleotiden, die in der Region 2p21 distal und proximal zur EML4-Bruchpunktregion gelegene Sequenzen gerichtet sind. Der Nachweis der Markierungen erfolgte über primäre (nicht markierte) Antikörper (Anti-DIG/Anti-DNP/Anti-BIO), die von sekundären polymerisierten Enzym-konjugierten Antikörpern (HRP-Polymer/AP-Polymer/beta-GAL) detektiert werden, sowie die enzymatische Umsetzung der Substrate (AP-RED/HRP-GREEN/beta-GAL-BLUE), die zur Entstehung starker, permanenter, roter, grüner und blauer Signale, die licht-mikroskopisch z.B. mit einer 40x-Trockenlinse dargestellt werden können, führt.

## Patentansprüche

1. Verfahren zur Detektion mehrerer unterschiedlicher Chromosomen- oder DNS-Bereiche in einer Zelle zum Nachweis von strukturellen Chromosomenaberrationen, wobei die Chromosomenaberrationen mindestens zwei Bruchpunktbereiche innerhalb eines Chromosoms aufweisen, basierend auf direkt oder indirekt markierten Nukleinsäurefragmenten (Sonden), **dadurch gekennzeichnet, dass**
- eine mit einem Label A markierte erste Sonde (Sonde A) und eine mit einem Label B markierte zweite Sonde (Sonde B) einen Bruchpunktbereich 1 flankieren, die Fusionssignale A-B bilden und
- zwei mit jeweils einem Label C markierten dritte und vierte Sonde (Sonden C) einen Bruchpunktbereich 2 flankieren, die Fusionssignale C-C bilden, wobei
die vorgenannten Fusionssignale sich bei einer Chromosomenaberration zu Fusionssignale A-C und Fusionssignale B-C verändern.

2. Verfahren nach Anspruch 1, **dadurch gekennzeichnet, dass** es sich bei den Sonden um Polynukleotide, modifizierte Polynukleotide oder modifizierte Nukleinsäurefragmenten oder Oligonukleotide oder modifizierte Oligonukleotide handelt.

3. Verfahren nach Anspruch 2, **dadurch gekennzeichnet, dass** die Sonden A, B und C unterschiedliche Label aufweisen sind.

4. Verfahren nach Anspruch 3, **dadurch gekennzeichnet, dass** das Label ausgewählt wird aus der Gruppe umfassend Farbstoffe, Farbstoffsubstrate, Chemielumineszenzfarbstoffe (z. B. Acridinium), Radioisotope, Spin-Label, Enzyme (z. B. Alkalische Phosphatase, Meerrettich-Peroxidase, Sojabohnen-Peroxidase und/oder beta-Galactosidase), Haptene (z. B. Dioxigenin, Biotin, 5(6)-Carboxyfluorescein, Rhodamin, Bromdeoxyuridin, Acetylaminofluoren, Trinitrophenol, Trinitrophenol - Derivat, Estradiol, und/oder DNP), Quantum Dots, Beads, Aminohexyle, Pyrene und Fluoreszenzfarbstoffe (z. B. Fluorecein, Fluorescein-Derivat, 5(6)-Carboxyfluorescein, Coumarin, Coumarin-Derivat, Rhodamin, Rhodamin-Derivat, Tetramethylrhodamin, Lissamin, Texas Red, AMCA, TRITC, IR Farbstoff, Alexa-Farbstoff, Dyomics-Farbstoff, Phycoerythrine, Cascade Blue, Oregon Green 488, Pacific Blue und/oder Rhodamine Green).

5. Verfahren nach Anspruch 3, **dadurch gekennzeichnet, dass** das Verfahren mittels der FISH-Methode unter Verwendung von drei direkt eingebauten Fluoreszenzfarbstoffen für die Emissionsbereiche Grün, Rot und Blau durchgeführt wird.

6. Verfahren nach Anspruch 3, **dadurch gekennzeichnet, dass** das Verfahren mittels der FISH-Methode unter Verwendung von drei direkt eingebauten Fluoreszenzfarbstoffen für die Emissionsbereiche Grün, Orange/Rot und Gold durchgeführt wird.

7. Verfahren nach Anspruch 3, **dadurch gekennzeichnet, dass** das Verfahren mittels der BrISH-Methode unter Verwendung von Biotin, Digoxigenin und DNP, die sich mit Antikörper-gekoppelter alkalischer Phosphastase, Antikörper-gekoppelter Peroxidase und Antikörper-gekoppelter beta-Galactosidase verbinden, durchgeführt wird.

8. Verfahren nach einem der vorgenannten Ansprüche zum Nachweis von Inversionen, Insertion und/oder Duplikation.

9. Verfahren nach einem der vorgenannten Ansprüche zum Nachweis von Inversionen, Insertionen und/oder Duplikationen kurzer genomischer Abschnitte.

10. Verfahren nach Anspruch 9, **dadurch gekennzeichnet, dass** invertierte, inserierte und/oder duplizierte genomische Abschnitte im Bereich kleiner als 20 Mb, bevorzugt kleiner als 15 Mb, besonders bevorzugt kleiner als 10 Mb und noch bevorzugter kleiner als 5 Mb nachgewiesen werden.

11. Verfahren nach Anspruch 8, **dadurch gekennzeichnet, dass** die geänderten Fusionssignale A-C oder B-C bei einem großen Bruchpunktbereich als Fusionssignal A-C und Signal C / Signal B oder als Fusionssignal B-C und Signal C / Signal A im Signalmuster sichtbar sind.

12. Verfahren nach Anspruch 9 zur Unterscheidung von Inversionen und Translokationen.

13. Verfahren nach Anspruch 8 zum Nachweis von Inversionen, wobei die Inversion inv(2)(p22-p21 p23) und/oder die Gene ALK und/oder EML4 betroffen sind.

14. Zubereitung zur Detektion mehrerer unterschiedlicher Chromosomen- oder DNS-Bereiche in einer Zelle zum Nachweis von strukturellen Chromosomenaberrationen, wobei die Chromosomenaberrationen mindestens zwei Bruchpunktbereiche innerhalb eines Chromosoms aufweisen, basierend auf direkt oder indirekt markierten Nukleinsäurefragmenten (Sonden), umfasst
- eine mit einem Label A markierte erste Sonde (Sonde A) und eine mit einem Label B markierte zweite Sonde (Sonde B) einen Bruchpunktbereich 1 flankieren, die Fusionssignale A-B bilden und
- zwei mit jeweils einem Label C markierten dritte und vierte Sonde (Sonden C) einen Bruchpunktbereich 2 flankieren, die Fusionssignale C-C bilden, wobei
die vorgenannten Fusionssignale sich bei einer Chromosomenaberration zu Fusionssignale A-C und Fusionssignale B-C verändern.

15. Zubereitung nach Anspruch 14, **dadurch gekennzeichnet, dass** die Sonden nach Ansprüchen 2 - 7 ausgestaltet sind.

## Claims

1. A method for the detection of multiple different chromosome or DNS regions in a cell for the detection of structural chromosome aberrations, wherein the chromosome aberrations have at least two breaking point regions within a chromosome, based on the directly or indirectly tagged nucleic acid fragments (probes), **characterized in that**
- a first probe (probe A) tagged with a label A, and a second probe (probe B) tagged with a label B, flanking a breaking point region 1, forming fusion signals A-B, and
- two third and fourth probes (probe C), each tagged with a label C, flanking a breaking point region 2, forming fusion signals C-C, wherein
with a chromosome aberration the fusion signals mentioned above convert into fusion signals A-C and fusion signals B-C.

2. The method according to claim 1, **characterized in that** the probes are polynucleotides, modified polynucleotides, or modified nucleic acid fragments, or oligonucleotides, or modified oligonucleotides.

3. The method according to claim 2, **characterized in that** probes A, B, and C have different labels.

4. The method according to claim 3, **characterized in that** the labels are selected from the group consisting of colorants, colorant substrates, chemiluminescence colorants (e.g., Acridinium), radioisotopes, spin-labels, enzymes (e.g., alkaline phosphatase, horseradish peroxidase, soybean peroxidase, and/or beta-galactosidase), haptene (e.g., dioxigenin, biotin, 5(6)-carboxy fluorescein, rhodamine, bromine deoxyuridine, acetyl amino fluorine, trinitrophenol, trinitrophenol derivative, estradiol, and/or DNP), quantum dots, beads, aminohexyle, pyrenes, and fluorescence colorants (e.g., fluorescein, fluorescein derivative, 5(6)-carboxy fluorescein, coumarin, coumarin derivative, rhodamine, rhodamine derivative, tetramethyl rhodamine, lissamin, Texas red, AMCA, TRITC, IR colorant, Alexa colorant, domes colorant, phycoerythrin, cascade blue, Oregon green 488, Pacific blue, and/or rhodamine green).

5. The method according to claim 3, **characterized in that** the method is carried out by means of the FISH method using three indirectly incorporated fluorescence colorants for the emission regions green, red, and blue.

6. The method according to claim 3, **characterized in that** the method is carried out by means of the FISH method using three indirectly incorporated fluorescence colorants for the emission regions green, orange/red, and gold.

7. The method according to claim 3, **characterized in that** the method is carried out by means of the BrISH method using biotin, digoxigenin and DNP, which form a bond with antibody-coupled alkaline phosphatase, antibody-coupled peroxidase, and antibody-coupled beta-galactosidase.

8. The method according to one of the previous claims for the detection of inversions, insertion, and/or duplication.

9. The method according to one of the previous claims for the detection of inversions, insertion, and/or duplication of genomic sections.

10. The method according to claim 9, **characterized in that** inverted, inserted, and/or duplicated genomic sections in the range of less than 20 Mb, preferably of less than 15 Mb, particularly preferred of less than 10 Mb, and most preferred of less than 5 Mb, are detected.

11. The method according to claim 8, **characterized in that** the converted fusion signals A-C or B-C are visible in the signal pattern at a large breaking point region as the fusion signal A-C and signal C/signal B, or as the fusion signal B-C and signal C/signal A.

12. The method according to claim 9 for differentiating inversions and translocations.

13. The method according to claim 8 for the detection of inversions, wherein the invention inv(2)(p22-p21 p23) and/or the genes ALK and/or EML4 are affected.

14. A preparation for the detection of multiple different chromosome or DNS regions in a cell for the detection of structural chromosome aberrations, wherein the chromosome aberrations have at least two breaking point regions within a chromosome, based on the directly or indirectly tagged nucleic acid fragments (probes), comprising
- a first probe (probe A) tagged with a label A, and a second probe (probe B) tagged with a label B, flanking a breaking point region 1, forming fusion signals A-B, and
- two third and fourth probes (probes C), each tagged with a label C, flanking a breaking point region 2, forming fusion signals C-C, wherein
with a chromosome aberration the fusion signals mentioned above convert into fusion signals A-C and fusion signals B-C.

15. The preparation according to claim 14, **characterized in that** the probes are embodied according to claims 2-7.

## Revendications

1. Procédé pour la détection de plusieurs domaines chromosomiques ou d'ADN différents dans une cellule, pour la détection d'aberrations chromosomiques structurelles, les aberrations chromosomiques comprenant au moins deux domaines points de rupture à l'intérieur d'un chromosome, sur la base de fragments d'acide nucléique (sondes) indirectement marqués, **caractérisé en ce que**
- une première sonde (Sonde A) marquée par un Marqueur A, et une deuxième sonde (Sonde B) marquée par un Marqueur B, flanquent un domaine point de rupture 1, formant les signaux de fusion A-B, et
- deux sondes, la troisième et la quatrième (Sondes C), chacune marquée par un Marqueur C, flanquent un domaine point de rupture 2, formant les signaux de fusion C-C,
les signaux de fusion mentionnés ci-dessus se transformant, en présence d'une aberration chromosomique, en des signaux de fusion A-C et des signaux de fusion B-C.

2. Procédé selon la revendication 1, **caractérisé en ce que**, pour ce qui concerne les sondes, il s'agit de polynucléotides, de polynucléotides modifiés ou de fragments d'acides nucléiques modifiés, ou d'oligonucléotides ou d'oligonucléotides modifiés.

3. Procédé selon la revendication 2, **caractérisé en ce que** les Sondes A, B et C comprennent des marqueurs différents.

4. Procédé selon la revendication 3, **caractérisé en ce que** le marqueur est choisi dans le groupe comprenant les colorants, les substrats de colorants, les colorants chimioluminescents (p.ex. d'acridinium), les radio-isotopes, les marqueurs de spin, les enzymes (p.ex. la phosphatase alcaline (p.ex. la phosphatase alcaline), la peroxydase du raifort, la peroxydase du soja et/ou la bêta-galactosidase), les haptènes (p.ex. la digoxigénine, la biotine, la 5(6)-carboxyfluorescéine, la rhodamine, la bromodésoxyuridine, l'acétylaminofluorène, le trinitrophénol, les dérivés du trinitrophénol, l'estradiol et/ou le DNP), les nanocristaux fluorescents, les perles, les aminohexyles, les pyrènes et les colorants fluorescents (p.ex. la fluorescéine, les dérivés de la fluorescéine, la 5(6)-carboxyfluorescéine, la coumarine, les dérivés de la coumarine, la rhodamine, les dérivés de la rhodamine, la tétraméthylrhodamine, la lissamine, le rouge Texas, l'AMCA, le TRITC, les colorants IR, les colorants Alexa, les colorants Dyomics, les phycoérythrines, le bleu Cascade, le vert Orégon 488, le bleu Pacific et/ou le vert de rhodamine).

5. Procédé selon la revendication 3, **caractérisé en ce que** le procédé est mis en oeuvre par la méthode FISH par utilisation de trois colorants fluorescents directement incorporés, pour les plages d'émission vert, rouge et bleu.

6. Procédé selon la revendication 3, **caractérisé en ce que** le procédé est mis en oeuvre par la méthode FISH par utilisation de trois colorants fluorescents directement incorporés pour les plages d'émission vert, orangé/rouge et or.

7. Procédé selon la revendication 3, **caractérisé en ce que** le procédé est mis en oeuvre par la méthode BrISH par utilisation de biotine, de digoxigénine et de DNP, qui s'assemblent à une phosphatase alcaline couplée à des anticorps, à une peroxydase couplée à des anticorps et à une bêta-galactosidase couplée à des anticorps.

8. Procédé selon l'une des revendications précédentes pour la détection d'inversions, d'insertions et/ou de duplications.

9. Procédé selon l'une des revendications précédentes, pour la détection d'inversions, d'insertions et/ou de duplications de segments génomiques courts.

10. Procédé selon la revendication 9, **caractérisé en ce que** les segments génomiques inversés, insérés et/ou dupliqués sont détectés dans la plage inférieure à 20 Mb, de préférence inférieure à 15 Mb, d'une manière particulièrement préférée inférieure à 10 Mb et d'une manière encore plus préférée inférieure à 5 Mb.

11. Procédé selon la revendication 8, **caractérisé en ce que** les signaux de fusion modifiés A-C ou B-C sont visibles, pour un grand domaine point de rupture, en tant que signal de fusion A-C et signal C-/signal B, et en tant que signal de fusion B-C et signal C/signal A dans le modèle de signal.

12. Procédé selon la revendication 9, pour distinguer les inversions et les translocations.

13. Procédé selon la revendication 8 pour la détection d'inversions, auquel cas il s'agit de l'inversion inv(2)(p22-p21p23) et/ou des gènes ALK et/ou EML4.

14. Préparation pour la détection de plusieurs domaines chromosomiques ou d'ADN différents dans une cellule pour la détection d'aberrations chromosomiques structurelles, les aberrations chromosomiques comprenant au moins deux domaines points de rupture à l'intérieur d'un chromosome, sur la base de fragments d'acides nucléiques directement ou indirectement marqués, comprenant :
- une première sonde (Sonde A) marquée par un Marqueur A, et une deuxième sonde (Sonde B) marquée par un Marqueur B, flanquent un domaine point de rupture 1, formant les signaux de fusion A-B, et
- deux sondes, la troisième et la quatrième (Sondes C), chacune marquée par un Marqueur C, flanquent un domaine point de rupture 2, formant les signaux de fusion C-C,
les signaux de fusion mentionnés ci-dessus se transformant, en présence d'une aberration chromosomique, en des signaux de fusion A-C et des signaux de fusion B-C.

15. Préparation selon la revendication 14, **caractérisée en ce que** les sondes sont configurées selon les revendications 2 à 7.
